# EUROPEAN PATENT SPECIFICATION

(11) **EP 3 468 568 B1**
(45) Date of publication and mention of the grant of the patent: **02.09.2020**
(21) Application number: 17727907.2
(22) Date of filing: 07.06.2017
(51) Int. Cl.: A61K 35/19, A61M 1/00, A61P 25/00, A61P 25/28, A61P 17/00, A61P 29/00

(54) **HUMAN PLATELET LYSATE DERIVED EXTRACELLULAR VESICLES FOR USE IN MEDICINE**
MENSCHLICHES BLUTPLÄTTCHENLYSAT ODER EINE FRAKTION, DIE FÜR MENSCHLICHES BLUTPLÄTTCHENLYSAT ANGEREICHERT IST, DIE VON EXTRAZELLULÄREN VESIKELN ZUR VERWENDUNG IN DER MEDIZIN ABGELEITET IST
LYSAT DE PLAQUETTES HUMAINES OU FRACTION ENRICHIE DE LYSAT DE PLAQUETTES HUMAINES DÉRIVÉES DE VÉSICULES EXTRACELLULAIRE POUR UTILISATION EN MÉDECINE

(30) Priority: 08.06.2016 EP 16173465
(43) Date of publication of application: 17.04.2019
(73) Proprietor: Lysatpharma GmbH, 07745 Jena (DE)
(72) Inventor: DE MIROSCHEDJI, Kyra Natalia Matahari, 07743 Jena (DE)
(74) Representative: DTS Patent- und Rechtsanwälte Schnekenbühl und Partner mbB
(86) International application number: PCT/EP2017/063868
(87) International publication number: WO 2017/211906

(56) References cited:
- EP-A1- 2 389 942
- WO-A1-2010/064267
- WO-A1-2014/013029
- WO-A2-2013/076507
- US-A1- 2012 156 306
- TORREGGIANI E ET AL: "EXOSOMES: NOVEL EFFECTORS OF HUMAN PLATELET LYSATE ACTIVITY", EUROPEAN CELLS & MATERIALS, vol. 28, July 2014 (2014-07), pages 137-151, XP002766230,
- TORREGGIANI E ET AL: "Isolation and characterization of exosomes derived from human platelet lysate", EUROPEAN CELLS AND MATERIALS 2013 AO RESEARCH INSTITUTE CHE, vol. 26, no. SUPPL. 3, 2013, page 64, XP002766231, ISSN: 1473-2262

## Description

### BACKGROUND

*Human* platelet lysate (*h*PL) is known as an alternative supplement for fetal bovine serum (FBS) in *human* cell culture. It is commonly used for supplementation of basal media in *human* mesenchymal stem cell culture for experimental and clinical purpose. *H*PL is advantageous compared to the traditionally used FBS as it is xenogen-free - and hence suitable for generation of therapeutic products for use in cell therapy. *H*PL appears as a turbid, light-yellow liquid that is obtained from lysis of *human* blood platelets by freezing/thawing cycle(s). By virtue of their physiological tissue repair function, platelets are a rich source of numerous bioactive molecules like growth factors, cytokines including chemokines and interleukins, other metabolites and extracellular vesicles (EV). During storage and lysis, the platelets release a large quantity of growth factors and extracellular vesicles (EVs e.g. exosomes, microvesicles, apoptotic bodies), ranging from approximately 40-1000 nm in size. These secreted vesicles are designated as *human* platelet lysate derived extracellular vesicles (*h*PLEVs). In the last years several *h*PL-related GMP products came onto the market aiming the use in cell culture systems, e.g. products from Stem Cell Technologies, Macopharma, COMPASS Biomedical, and PL BioSciences. Recently, the Center for Clinical Transfusion Medicine in Tübingen has obtained a manufacturing license for *human* platelet lysate in accordance with the German Drug Law (AMG) by the local governmental office of Tübingen. Such *h*PL products are required for the cultivation of stem cells according to the regulatory requirements for their use in *humans.* Meanwhile *h*PL is the gold standard for cultivation of *human* stem cells, especially mesenchymal stem cells. *Human* platelet lysate obtained under GMP cleanroom-conditions is considered as "raw material of *human* origin" for pharmaceutical manufacturing - such as for use in clinical trials or for manufacturing-licensed medicinal products for cell therapy.

Although the value of *h*PL in cultivation of stem cells is acknowledged, the properties of the single factors in the *h*PL such as growth factors and EVs are not yet analyzed in detail. While EVs from *h*PL have not been considered to be of clinical interest, EVs derived from different stem cells, such as MSCs, are increasingly in the focus of clinical and therapeutic interest.

EVs are known to transmit information between cells, organs and even between organisms, and have been detected in various body fluids, such as blood, urine, cerebrospinal liquid, breast milk and saliva. Exosomes and microvesicles comprise the most prominently described classes of EVs. They are surrounded by a phospholipid membrane and contain cell-type-specific combinations of proteins, including enzymes, growth factors, receptors and cytokines as well as lipids, coding and non-coding RNAs, mRNA, microRNA (miRNA), or even small amounts of DNA, and metabolites. Exosomes are defined as 70-170 nm sized (+/- 20 nm, this varies depending on literature and technique of analysis) derivatives of the endosomal compartment. With average sizes of 100-1000 nm, microvesicles represent a class of larger EVs that are formed by the outward budding of the plasma membrane. In the present application the term EV comprises all of the above mentioned vesicles. For now it seems that only stem cell derived EVs are widely considered and discussed to have therapeutic potential according to therapeutic purposes. WO 2012/053976 discloses the use of exosomes that are secreted by *human* mesenchymal stem cells in order to promote hair-growth and wound-healing. These effects are disclosed in connection with the exosomal immunomodulatory cargo. WO 2012/053976 speculates about an immunomodulatory effect of the exosome-preparation.

WO 2014013029 A1 relates to the use of exosome-preparations derived from neonatal or adult tissue-derived mesenchymal stem-cells (MSCs) for prevention or therapy of inflammatory conditions, such as pre- and postnatally acquired damages of the brain (i.e. neuronal damages) or immunologic complications following stem cell transplantation (*"Graft versus Host-Disease",* GvHD).

The latest position-paper published by ISEV (The International Society of Extracellular Vesicles) in the Journal of Extracellular Vesicles by Thomas Leneret et al., 2015 (Applying extracellular vesicles based therapeutics in clinical trials. 4: 30087) describes recently discussed sources of EVs with therapeutic potential. Cell sources under investigation for regenerative medicine are endothelial cells and endothelial colony forming cells, including *human* endothelial cells from umbilical vein and late endothelial cells. In addition, haematopoietic progenitors that are capable of differentiating into myeloid and lymphoid cells may exert pro-angiogenic functions. Neural stem cells (NSCs) have been used in preclinical models in a variety of neurologic and neuroinflammatory disorders such as stroke, multiple sclerosis (MS) or spinal cord injuries. It became evident, however, that analogously to MSCs also NSCs exert their therapeutic effects in a paracrine and systemic manner rather than by migrating into sites of lesion. In this context, NSC-derived EVs are considered to interact with the host's immune-system to mediate neuroprotection and immunomodulation. Neuroprotection and neuroregeneration can also be mediated by EVs released from the resident glia cells of the nervous system. Finally, very recent studies describe the isolation of EVs from induced pluripotent stem cells (iPSCs), their ability to transfer RNAs and proteins into heart-cells, and their healing abilities *in vivo* in ischaemic myocardia. In addition, iPSCs might be used as a source to raise somatic stem cells in a scaled manner for the large-scale EV-production or to obtain cells as an EV source that can hardly be obtained from primary donor material, such as *human* NSCs. In this context, EVs from iPSC-derived MSCs have already been shown to attenuate limb-ischaemia. It is discussed that the combination of iPSC and EV-technologies might provide novel therapeutic options in the future.

To overview the given in the above mentioned recent position paper (Journal of Extracellular Vesicles 2015, 4: 30087) it has to be underlined that the therapeutic potential of EVs deriving from *h*PL is unconsidered.
EVs deriving from *human* stem cells such as MSCs, NSCs or iPSCs are not easily accessible as the stem cell cultivation is complex, expensive and sources are limited for pharmaceutical large-scale manufacturing.

In a publication from Torreggiani et al. (2014, European Cells and Materials Vol.28, 137-151) it is discussed that *h*PL-derived exosomes might be considered as novel effectors in *human* platelet lysate activity. Torreggiani *et al.* discuss the use of the platelet-derived exosomes for bone regeneration. In their study the role of PL-derived exosomes in connection with MSC-cell culture support was investigated. However, the preparation described in Torreggiani *et al.* does not seem to fulfill the standard of quality for a reliable extraction of EVs.

In addition, in most clinical studies using MSC-derived EVs, MSCs are cultured in *h*PL-supplemented media, wherein potential synergistic effects of *hPLEV*s are not addressed or discussed. In the prior art the effects observed in MSC-derived EV studies are attributed to the MSC-derived-EVs even though *h*PL was used as supplementation of basal media in mesenchymal stem cell cultures.

US20120156306A1 relates to a viral-safe platelet extract, to its preparation and use.

EP2389942A1 relates to a virally-inactive growth factors containing platelete lysate depleted of PDGF and VEGF and preparation and method thereof.

WO2010064267A1 relates to platelets lysates and bioadesive compositions thereof for the treatment of mucositis.

WO2014013029A1 relates to the use of preparations comprising exosomes derived from mesenchymal stem cells (MSCS) in the prevention and therapy of inflammatory conditions.

Moreover, there are numerous scientific articles, patent applications and patents on the use of Platelet Rich Plasma (PRP) as e.g. Eppley BL et al. (2006) in Plast Reconstr Surg 118(6): 147e-159e, Mishra AK et al. (2012) in Curr Pharm Biotechnol 13(7):1185-1195 and Carlson NE et al. (2002) in J Am Dent Assoc 133(10):1388-1386 . PRP consists of concentrated living platelets and plasma derived from a patient's whole blood, centrifuged to remove red blood cells and other unwanted components. It has a greater concentration of growth factors than whole blood and has been used in tissue injections in a variety of disciplines, including dentistry, orthopedic surgery, and sports medicine.

However, until now in 2016, results of basic science and preclinical trials have not yet been confirmed in large-scale randomized controlled trials. In a review from 2009 scientific literature was screened systematically and there were only few randomized controlled trials found, that adequately evaluated the safety and efficacy of PRP treatments. PRP was concluded to be "a promising, but not proven, treatment option for joint, tendon, ligament, and muscle injuries" (see also Foster et al. (2009). Am J Sports Med 37 (11): 2259-72).

Regarding the use of PL there have been described particular applications in the field of wound healing as in WO20130765507 which describes a pharmaceutical composition comprising a platelet lysate and its use to treat a wound, an anal fissure, vaginal atrophy or a wrinkle. In Fontana et al. (2016) ASC Appl Mater Interfaces 8(1): 988-996 PL-modified silicon microparticles for enhanced cell proliferation in wound healing applications are described.

Summarizing, it seems that in PRP-related prior art, the use of *h*PL or *h*PLEVs for clinical applications beyond wound healing applications is not described.

Concerning the art, there is thus a critical need for means and methods that allow performing easy, cheap, reliable and efficient therapies using PRP and PL as a source. Same pertains for EV-based therapies that are not based on complex and expensive stem cell culture systems.

The object of the present invention is to comply with the needs mentioned above and present *h*PL-derived EVs as novel tool in medicine, particularly in therapeutic, regenerative and preventive medicine.

### SUMMARY

The present invention is related to a pharmaceutical preparation comprising *human* platelet lysate or a fraction that is enriched for *human* platelet lysate derived extracellular vesicles (this fraction is also abbreviated *h*PLEVs in the following) for use in medicine, particularly for the prevention and/or treatment of acute or/and chronic inflammatory diseases and immune disorders (also including autoimmune diseases and diseases resulting from transplant rejections e.g. *GvHD*), neurologic and neurodegenerative diseases (stroke, ischaemia), dermatologic diseases, cardiovascular diseases, orthopaedic diseases, infectious diseases, cancer-diseases, tissue-regeneration (solid organs, hollow structures, injuries).

The present invention is further related to cosmetic applications of *human* platelet lysate or a fraction that is enriched for *human* platelet lysate derived extracellular vesicles. These applications include: cosmetic skin related anti-inflammatory treatments, skin regeneration after injury or burn, skin anti-aging therapies, as well as prevention and treatment of hair loss.

The present invention relates also to diagnostic applications of *human* platelet lysate or a fraction that is enriched for *human* platelet lysate derived extracellular vesicles.

The present invention is further related to a method of manufacturing of a pharmaceutical preparation or a diagnostic preparation or a cosmetic preparation comprising the step of adding *human* platelet lysate or a fraction that is enriched for *human* platelet lysate derived extracellular vesicles to the pharmaceutical preparation or a diagnostic preparation or a cosmetic preparation.

Although there are a number of advantages in terms of biological importance, costs and efforts by substituting stem cell derived EVs by *h*PLEVs, such a substitution was never suggested in the prior art. As *h*PL derives from *human* blood/plasma the source is regenerative and easy to access in contrast to MSCs, ESCs, NSCs or other stem cell types. Thus *h*PLEVs are easier accessible and less expensive than EVs deriving from stem cell cultures. Further advantage of the present invention over corresponding stem cell culture-derived EVs is the fact that platelets do not require clean room conditions and that no time-consuming and expensive flow cytometric characterization of the cellular entities are required. Additionally, excess *human* platelet concentrate-products, which were produced for application in hospitals and clinics and are expired after several days, can be still used for production of *h*PL instead of discarding them.

Furthermore, the invention is related to *h*PL which is produced out of expired, frozen and stored platelets, particularly not later than seven days after collection, providing the active components of the platelets immediately and not in the retarded release way of living platelets. Thus, one important aspect of the present invention is related to the use of *h*PL- or *h*PLEV for a medical or cosmetic treatment instead of using showing a surprising advantage over the use of living platelets, e.g. platelet rich plasma therapy, a *h*PL- or *h*PLEV-therapy is of surprising advantage.

Of note, the present invention describes for the first time to directly use *human* platelet lysate or a fraction that is enriched for *human* platelet lysate-derived extracellular vesicles in medicine particularly for the prevention and/or treatment of acute or/and chronic inflammatory diseases and immune disorders (also including autoimmune diseases and diseases resulting from transplant rejections e.g. *GvHD*), neurologic and neurodegenerative diseases (stroke, ischaemia), dermatologic diseases, cardiovascular diseases, orthopaedic diseases, infectious diseases, cancer-diseases, tissue-regeneration (solid organs, hollow structures, injuries) and diseases where a antimicrobial treatment is of advantage.

The present invention teaches for the first time to directly use *human* platelet lysate or a fraction that is enriched for *human* platelet lysate-derived extracellular vesicles as a medicament and for cosmetic applications. These applications include cosmetic skin related anti-inflammatory treatments, antimicrobial treatments, skin regeneration after injury or burn, skin anti-aging therapies, as well as prevention and treatment of hair loss.

The present invention presents for a first time the novel use of *h*PL or *h*PLEVs as medicament, which can be manufactured at low costs, is a xenogeny and cell-free product of highly medical quality and whereas no clinical cell culture intermediate is required..

### DETAILED DESCRIPTION

To support the understanding of the invention, several terms are defined below. Unless defined otherwise, all technical and scientific terms used herein have the same meaning as commonly understood by one of skill in the art. Although any methods and materials similar or equivalent to those described herein can be used in the practice or testing of the claims, the exemplary methods and materials are described herein.

Moreover, reference to an element by the indefinite article "a" or "an" does not exclude the possibility that more than one element is present, unless the context clearly requires that there be one and only one element. The indefinite article "a" or "an" thus usually means "at least one."

The term "about" means within a statistically meaningful range of a value or values such as a stated concentration, length, molecular weight, pH, time frame, temperature, pressure or volume. Such a value or range can be within an order of magnitude, typically within 20%, more typically within 10%, and even more typically within 5% of a given value or range. The allowable variation encompassed by "about" will depend upon the particular system under study.

The terms "comprising," "having," "including," and "containing" are to be construed as open-ended terms (i.e., meaning "including, but not limited to,") unless otherwise noted.

Recitation of ranges of values herein are merely intended to serve as a shorthand method of referring individually to each separate value falling within the range, and includes the endpoint boundaries defining the range, unless otherwise indicated herein, and each separate value is incorporated into the specification as if it were individually recited herein.

In context of the present invention the *h*PL means any kind/pool of *human* platelet lysate which has a beneficial therapeutic or cosmetic effect. Platelets, also named thrombocytes, are irregular, disc-shaped elements in the blood, that assist in blood-clotting. During normal blood-clotting, the platelets agglutinate by aggregation. Although platelets are often classified as blood-cells, they have no nucleus. They derive from large cells called megakaryocytes located/sited in bone marrow. For production of *h*PL, platelets are lysed and by this release their inner content into the surrounding plasma. Lysis of platelets can be achieved by freeze and thaw cycles. Suitable protocols can be found in the prior art.

One biologic component that is contained to a high content in *human* platelet-lysates is EVs. As EVs are present in most body fluids, plasma-derived EVs may originate from different cell-types, such as leucocytes, erythrocytes, dendritic cells (DCs), platelets, mast cells, epithelial cells, endothelial cells, and neurons. On one side, *h*PL may contain plasma-derived EVs that were already present in the blood plasma at the moment of blood-donation. On the other side, *h*PL might contain high amounts of specific platelet-derived EVs, secreted by living thrombocytes during storage time of platelet-concentrate-products. During the storage time of nearly one week at 20-24°C, cells continue secreting their specific EVs into the surrounding plasma. When the expiry date of thrombocyte-concentrates is reached and blood-products can be used for other purposes like the production of platelet-lysate-products, this specific-EV-enrichment should be still present in the lysate which can be further processed to gain *h*PLEVs enriched fractions. Additionally, platelets burst by lysis and release their soluble inner components into the plasma.

Protein-profiles of EVs differ according to their cellular origin.

Specific CD markers for the characterization of platelets are the surface markers CD9, CD41b, CD42a, CD42b and CD61 that appear on the platelet surface before activation.

There are also markers that appear on the platelet surface during activation such as PAC-1, CD62P, CD31, CD63 and Syntenin
Exosomes from platelets or endothelial cells can be for example identified by the expression of typical surface markers (e.g. CD31 = platelet endothelial cell adhesion molecule-1 or CD62P = P-selectin). These markers correspond to surface markers on the secreting cell-entities. Plasma-EVs may derive from different cell-subpopulations and therefore may also contain different marker-subsets.

Another point is that *h*PL contains EVs that were released from living thrombocytes during the period of storage of thrombocyte-concentrates (20-24°C) before reaching the expiry date after 4-6 days. Thus the *h*PL contains a significant fraction of EVs originating from *human* platelets. Compared to normal plasma-products, platelet-lysates produced from expired thrombocyte-concentrates are highly enriched for platelet-EVs. At time point of lysis with processing afterwards, all soluble, paracrine factors of the concentrates are saved while getting rid of cells and other cellular components.

EVs function as a vehicle for various biomolecules including lipids, proteins (e.g. transcription factors, cytokines, growth factors) and nucleic acids such as mRNA, microRNA (miRNA) or even small amounts of DNA.

Lipid components of exosomes include membrane lipids such as sphingomyelin, phosphatidylcholine, phosphatidylethanolamine, phosphatidylserine, gangliosides (GM3), phosphatidylinositol, prostaglandins and lysobisphosphatidic acid.

Furthermore, in addition to lipids and proteins, exosomes also may contain nucleic acids including mRNA, miRNA and a large variety of other small noncoding RNA species, including RNA transcripts overlapping with protein coding regions, repeat sequences, structural RNAs, tRNA, rRNA, vault RNA, Y RNA, and small interfering RNAs (siRNA) as well as mitochondrial DNA, and short DNA sequences of retrotransposons.

The term "nucleic acid" as used herein generally comprises polyribonucleotides (RNA) and polydeoxyribonucleotides (DNA), each in single-stranded and/or doublestranded form, linear or circular, or mixtures thereof, including hybrid molecules. RNAs may include, without limitation, messenger RNAs (mRNA), non-coding (ncRNAs-including anti-sense-RNAs), silencer RNAs, micro-RNAs (miRNAs), short hairpin RNAs (shRNAs), small interfering RNAs (siRNAs), repeat-associated small interfering RNA (rasiRNA), piwi-interacting RNAs (piRNA), Y RNA, Long non-coding RNAs (long ncRNAs, IncRNA)), transfer RNAs (tRNA), ribosomal RNAs (rRNA), small nuclear RNA (snRNA), small nucleolar ribonucleic acid (snoRNA), spliced leader RNAs (SL RNA). All of the aforementioned RNAs are in principle conceivable as EV constituents and may be utilized within the methods of the present invention.

EVs also comprise proteins and peptides. The terms "polypeptide" and "protein" are used interchangeably herein. The term "polypeptide" refers to a protein or peptide that typically contains or consists of at least 20, and preferably at least 30, such as at least 50 amino acids. The term "peptide" refers to an oligomer containing or consisting of at least 2 amino acids to about 19 amino acids.

The most frequently identified proteins of EVs are membrane transporters and fusion-proteins (e.g., GTPases such as Rab5, annexins, and flotillins), heat shock proteins (e.g., HSC70), tetraspanins (e.g., CD9, CD63, and CD81), proteins from MVB-biogenesis (e.g., Alix and TSG101), lipid-related proteins and phospholipases, cytoskeleton proteins (actins, cofilin-1, ezrin/radixin/moesin, profilin-1, and tubulins), metabolic enzymes, and ribosomal proteins. Several proteins are recognized as exosomal markers, among which the tetraspanins (e.g. CD63, CD81) and TSG101, a protein of the ESCRT-complex, are the most commonly used markers for detection.

Although the latter are commonly used as markers of exosomes, they may not be exclusive to exosomes and may be found on other extracellular vesicles.

In context of the present invention, *h*PL comprises any *human* platelet lysate that might be useful in context of therapeutic, diagnostic or cosmetic applications. Preferably it is *h*PL produced according to GMP-conditions and preferably it has been manufactured in accordance with the German Drug Law (AMG). The origin of the *h*PL might originate from single or pooled donor-donated platelets. It might be preferred to use *h*PL originating from blood donors at certain age, e.g. from blood donors between 10-60 years, 18-50 years, 18-40 years, 18-30 years, or 18-20 years. In the context with precision medicine it might be of advantage to treat a patient with *human* platelet lysate or a fraction that is enriched for *human* platelet lysate derived extracellular vesicles originating from its own blood donation. According to a preferred embodiment of the present invention the *h*PL originates from healthy individuals.

To avoid misunderstandings, the *h*PL is generally enriched for *human* platelet-derived extracellular vesicles compared to plasma-EVs before any EV-enrichment, isolation or purification method. In the context of the present invention the term "fraction that is enriched for *human* platelet lysate-derived extracellular vesicles" means that the *h*PL was processed by at least one step of EV-enrichment, isolation or purification method. After this enrichment, isolation or purification step the "fraction that is enriched for *human* platelet lysate derived extracellular vesicles" contains less non-EV contaminants.

*H*PL or a fraction that is enriched for *h*PL can be obtained from platelets that have been incubated before the lysis with one or more compounds known as thrombocyte activators from the prior art, e.g. prior art related to PRP therapy, that activate the platelets and thus improve the quality of the inventive preparation.

According to one embodiment of the invention *h*PL might be derive from a Human Umbilical Cord Blood. Human Umbilical Cord Blood Platelet Lyse preparations are known from the prior art (e.g. US 8501170 B2, Parazzi, V., C. Lavazza, et al. (2015) or Forte, D., M. Ciciarello, et al. (2015).

For the purposes of the present invention, the terms "isolation" and "isolating" in all their grammatical forms relate to the act of separating or recovering EVs from their environment, e.g. a serum or plasma sample. The terms "purifying" and "purification" in all their grammatical forms relate to the act of (substantially) reducing/depleting (non-EV) contaminants from the desired EVs. The terms "enriching" and "enrichment" in all their grammatical forms mean increasing the proportion of EVs in their respective solvent(s).

The presently described *h*PL or a fraction that is enriched for *h*PLEVs can be used in medicine. According to one embodiment of the present invention, *h*PL or a fraction that is enriched for *h*PLEVs are used for the prevention and/or treatment of inflammatory driven diseases. Inflammatory abnormalities comprise a large group of disorders that underlie a vast variety of *human* diseases. The immune system is often involved with inflammatory disorders, demonstrated in both allergic reactions and some myopathies, with many immune system disorders resulting in abnormal inflammation. Non-immune diseases with etiological origins in inflammatory processes include cancer, arteriosclerosis, and ischaemic heart disease. A large variety of proteins is involved in inflammatory processes. Anyone of them might be modified due to genetic mutations resulting in impairments or dysregulations of the normal protein function or protein expression. Examples of disorders associated with inflammation include: acne vulgaris, asthma, autoimmune diseases, celiac disease, chronic prostatitis, glomerulonephritis, hypersensitivities, inflammatory bowel diseases, pelvic inflammatory disease, reperfusion injury, rheumatoid arthritis, sarcoidosis, transplant rejection, vasculitis, and interstitial cystitis. Many diseases are considered to go along with inflammation or are categorized as autoimmune-diseases. Some various types of inflammatory diseases include: gout, lupus, asthma, pleurisy, eczema, arthritis, gastritis, splenitis, sinusitis, hepatitis, nephritis, psoriasis, vasculitis, laryngitis, thyroiditis, prostatitis, pharyngitis, sarcoidosis, atherosclerosis, allergic reactions, multiple sclerosis, some myopathies, rheumatoid arthritis, seborrheic dermatitis, Wegener's granulomatosis, irritable bowel syndrome (IBS; Crohn's disease), ulcerative colitis, diverticulitis.

According to one embodiment of the present invention, *h*PL or a fraction that is enriched for *h*PLEVs are used for the prevention and/or treatment of neurodegenerative diseases, e.g. but not limited to Alzheimer's Disease, amyotrophic lateral sclerosis, corticobasal degeneration, frontotemporal dementia, HIV-related cognitive impairment, Huntington's disease, Lewy body dementias, mild cognitive impairment, posterior cortical atrophy, primary progressive aphasia, progressive supranuclear palsy and vascular dementia.

According to one embodiment of the present invention, *h*PL or a fraction that is enriched for *h*PLEVs are used for the prevention and/or treatment of immune/autoimmune diseases e.g. but not limited to Addison disease, celiac disease - sprue (gluten-sensitive enteropathy), dermatomyositis, graves disease, Hashimoto thyroiditis, multiple sclerosis, myasthenia gravis, pernicious anemia, reactive arthritis, rheumatoid arthritis, Sjogren syndrome, systemic lupus erythematosus and type I diabetes.

According to one embodiment of the present invention, *h*PL or a fraction that is enriched for *h*PLEVs are used for the prevention and/or treatment of cardiovascular diseases, e.g. but not limited to coronary heart disease, cerebrovascular disease, peripheral arterial disease, rheumatic heart disease, congenital heart disease, deep vein thrombosis and pulmonary embolism.

According to one embodiment of the present invention, *h*PL or a fraction that is enriched for *h*PLEVs are used for the prevention and/or treatment of dermatologic diseases, e.g. but not limited to acne, eczema (atopic eczema), fungal infections of nails, herpes and psoriasis.

According to one embodiment of the present invention *h*PL or a fraction that is enriched for *h*PLEVs are used for the prevention and/or treatment of orthopedic diseases, e.g. but not limited to rheumatoid arthritis, bursitis, elbow pain and problems, cubital tunnel syndrome, lateral epicondylitis (tennis elbow), medial epicondylitis (golfer's or baseball elbow), fibromyalgia, foot pain and problems, fractures, hip fracture, low back pain, hand pain and problems, carpal tunnel syndrome, knee pain and problems, ligament injuries to the knee, torn meniscus, kyphosis, neck pain and problems, osteoporosis, paget's disease of the bone, scoliosis, shoulder pain and problems, soft-tissue Injuries.

According to one embodiment of the present invention, *h*PL or a fraction that is enriched for *h*PLEVs are used for the prevention and/or treatment of tissue regenerative medicine. *Tissue engineering* evolved from the field of biomaterial development and refers to the practice of combining scaffolds, cells and bioactive molecules into functional tissues. The goal of tissue-engineering is to assemble functional constructs that restore, maintain or improve damaged tissues or whole organs. Artificial skin and cartilage are examples of engineered tissues that have been approved by the FDA, however, they currently have limited use in *human* patients. Regenerative medicine is a broad field that includes tissue engineering but also incorporates research on self-healing - where the body uses its own systems to recreate cells and rebuild tissues and organs, sometimes supported by foreign/allogeneic biological material. The terms "tissue-engineering" and "regenerative medicine" have become largely interchangeable, as the field hopes to focus on cures instead of treatments for complex and often chronic diseases.

According to a further embodiment of the present invention, *h*PL or a fraction that is enriched for *h*PLEVs are used for the prevention and/or treatment of oncologic diseases, e.g. but not limited to bladder cancer, bone cancer, breast cancer, colon/rectum cancer, Hodgkin disease, leukemia, liver cancer, lung cancer, lymphoma of the skin, multiple myeloma, nasopharyngeal cancer, Non-Hodgkin lymphoma, osteosarcoma, ovarian cancer, pancreatic cancer, prostate cancer, sarcoma - adult soft tissue cancer, skin cancer, small intestine cancer, and stomach cancer.

According to a further embodiment of the present invention, *h*PL or a fraction that is enriched for *h*PLEVs are used for the prevention and/or treatment of transplant rejections. According to a further embodiment of the present invention, *h*PL or a fraction that is enriched for *h*PLEV are used for the prevention and/or treatment of stroke, ischemia or Graft-versus-Host Disease.

According to a preferred embodiment, the disease may be selected from the group of diseases that are currently treated by cell-therapy, e.g. but not limited to applications of allogeneic cell therapy, *human* embryonic stem cell therapy, neural stem cell therapy, mesenchymal stem cell therapy and hematopoietic stem cell transplantation applications.

Allogeneic cell therapies attempt to develop such products to treat conditions including Crohn's disease and a variety of vascular conditions. *Human* embryonic stem cell research has been used as the basis for a number of therapeutic applications, including possible treatments for diabetes and Parkinson's disease. In Neural Stem Cell Therapies, neural stem cells (NSCs) are the subject of ongoing research for possible therapeutic applications, for example for treating a number of neurological disorders such as Parkinson's disease and Huntington's disease. Mesenchymal stem cell therapy is used for a wide range of treatments including immunomodulatory therapies, bone and cartilage regeneration, myocardium regeneration and the treatment of Hurler syndrome, a skeletal and neurological disorder.
According to a further embodiment of the present invention *h*PL or a fraction that is enriched for *h*PLEV can be used for the prevention or treatment of infectious diseases, particularly caused by bacteria, viruses, fungi or parasites. One preferred embodiment is the treatment of infectious diseases in dermatology, such as cellulitis, erysipelas, hidradenitis suppurativa, impetigo and ecthyma, lymphadenitis, lymphangitis, necrotizing skin infections or staphylococcal scalded skin syndrome. One further preferred application of the inventive preparation is the application in context of indications in which a platelet-treatment or a platelet rich plasma-treatment is described in the prior art to have a positive effect. From the prior art it is known, that there is a growing appreciation of important immune- and inflammation-related functions of platelets, in both health and disease. A number of studies have demonstrated that platelets' impact in inflammatory-processes is ranging from atherosclerosis to infectious diseases, making platelets the most numerous circulating cell type that has an immunologic function. Platelets interact with white blood cells and vascular endothelial cells, directly by contact-dependent mechanisms and indirectly by mechanisms of secreted immune-mediators. Therefore, platelet mediated immune-effects occur locally at sites of platelet activation and deposition, or systemically at locations distant from platelet activation itself.

Platelets are best known as the cellular mediator of thrombosis (see Craig N. Morrell et al. 2014. "Emerging roles for platelets as immune and inflammatory cells" Blood: 123 (18)). In this article it is described that there is now a growing appreciation of the important immune and inflammatory roles of platelets in both health and disease. A number of studies have demonstrated that platelets impact on inflammatory processes are ranging from atherosclerosis to infectious diseases, making platelets the most numerous circulating cell type that has an immune function. Platelets interact with white blood cells and vascular endothelial cells both directly by contact-dependent mechanisms and indirectly through secreted immune mediator-driven mechanisms. Platelet immune effects are therefore noted both locally at sites of platelet activation and deposition or systemically at locations distant from platelet activation itself. Craig N. Morrell *et al.* conclude that platelet interactions with inflammatory cells may mediate proinflammatory outcomes, but these interactions have likely evolved to be beneficial in limiting infection. For example, with a breach in the skin there is exposure to pathogens, and by combining thrombotic and immune recruitment functions, platelets may help focus hemostasis and immune responses against potential infectious agents to prevent pathogen invasion. However, continued or chronic platelet interactions with white blood cells or endothelial cells can lead to adverse effects from excessive immune stimulation and inflammatory insult, see Craig N. Morrell *et al.* (2014). It is known from the prior art that platelets have a positive effect in context of axon-regeneration, wound-healing, and pain-reduction (Kuffler DP et al. in Mol Neurobiol. 2015 Oct; 52(2):990-1014).

According to the present invention it has been found that the important immune and inflammatory roles of platelets can be in part substituted and the regenerative properties can be improved by the use of hPL or hPLEV instead of the living platelets.

Preferred indications comprise thus wound-healing, tissue-regeneration, nerve-injury, tendinitis, osteoarthritis, cardiac muscle injury, bone-repair and regeneration and also plastic surgery and oral surgery.
According to a further embodiment of the present invention the preparation is cell-free. Cell-free in context of the present invention means that the preparation is substantially free of living intact cells and cell-fragments. The inventive *h*PLEV-fractions are preferably cell-free preparations, that are enriched for EVs while other components are reduced.

According to a further embodiment of the present invention, the *h*PL or the fraction that is enriched for *h*PLEVs is the essential pharmaceutically active ingredient in the preparation.

Being the essential pharmaceutically active ingredient means that the *h*PL or the fraction that is enriched for *h*PLEVs possesses an therapeutic or another value when administered to a *human* being. Being the essential pharmaceutically active ingredient means also that the preparation is substantially free of other pharmaceutically active ingredients except of the *human* platelet lysate, *h*PL-components or the fraction that is enriched for *human* platelet derived extracellular vesicles.

The preparation according to the present invention might comprise one or more excipients beside the *human* platelet lysate or the fraction that is enriched for *human* platelet lysate-derived extracellular vesicles. Such an excipient might be a natural or synthetic substance formulated alongside the active ingredient, for example for the purpose of long-term stabilization, bulking up solid formulations that contain potent active ingredients (thus often referred to as "bulking agents," "fillers," or "diluents"), or to confer a therapeutic enhancement on the active ingredient in the final dosage form, such as facilitating drug-absorption, reducing viscosity or enhancing solubility. Excipients can also be useful in the manufacturing process, to aid in the handling of the active substance concerned such as by facilitating powder-flow ability or non-stick properties, in addition to aiding *in vitro* stability such as prevention of denaturation or aggregation over the expected shelf life. The selection of appropriate excipients also depends upon the route of administration and the dosage form, as well as the active ingredient and other factors.

According to a further embodiment of the present invention the extracellular vesicles of the fraction that is enriched for *human* platelet lysate derived extracellular vesicles (exosomes) have a size of between about 70 to 200 nm, preferably between about 70 to 140 nm, or more preferably between about 70 to 120 nm. "About" shall mean a +/- 20% deviation. Further preferred, the exosomes are positive for characteristic EV- or exosome-markers, and even further preferred, the protein content of the pharmaceutical preparation is higher than 0.5 mg/ml, preferably higher than 1 mg/ml.

*h*PLEV markers comprise heat shock proteins (e.g., HSC70), tetraspanins (e.g., CD9, CD10, CD26, CD53, CD63, CD82), proteins from MVB-biogenesis (e.g., Alix and TSG101), EpCAM or Rab5, but substantially lacking CD81 (which is usually a general EV surface marker), CD2, CD8, CD11a and CD25 (Koliha *et al*, 2016).

According to a further embodiment of the present invention the extracellular vesicles are positive for at least one of the EV- or exosome-markers consisting of the group: CD9, CD41a, CD41b, CD42b, CD61, CD62P and CD63. Preferably the extracellular vesicles are positive for 2, 3, 4, 5, 6 or 7 of the above mentioned EV- or exosome-markers.

Being positive according to the present invention means that the background medium fluorescence intensity of one of the above mentioned positive surface markers when carrying out a multiplex bead-based platform analysis according to the method described in Koliha, Nina *et al*. (2016) is higher than a comparison example with extracellular vesicles deriving from NK cells.

Alternatively other techniques for specific protein detection of above mentioned markers can be applied, such as Western Blot Analysis.

According to a further embodiment of the present invention the extracellular vesicles are negative for at least one of the surface-markers consisting of the group CD81, CD3, CD4, CD19, CD20, CD2, CD8, CD11a and CD25. Preferably the extracellular vesicles are negative for 2, 3, 4, 5, 6, 7, 8 or 9 of the above mentioned cellular exosome markers.

To prove for the quality of *h*PLEV-enriched fractions, several general characterization criteria on the biologic and biophysical properties of contained EVs should be fulfilled. State of the art for such characterization is the criteria and standards recommended by the International Society for Extracellular Vesicles (ISEV). Based on the latest scientific knowledge in the EV-field these criteria should be used to attribute any specific biological cargo or function to EVs.

### Basic quality criteria/standards:

1. Determination of protein content [mg/ml] by standard protein quantification methods using BCA-assays, similar assays like the Bradford-Assay, or instruments based on spectrometry (like the "NanoDrop").
2. Determination of mean particle size [nm] and size distribution [curves] using the Nanoparticle Tracking Analysis (NTA) platform, such as Nanosight and Zetaview, or Image-Stream Flow Cytometry, such as "Amnis" that allows analysis of EVs at a single particle level.
3. Semiquantitative detection of typical EV-marker proteins including EV- or exosomal proteins (SDS-PAGE, Western Blot, detection with specific antibodies, detection of the signal). In general, the EV content is highly dependent on the cellular origin, on pre-conditioning of producing cell-lines and on the preparation method. However, EVs such as exosomes present a common set of generally expected markers that can be used for their characterization. Most commonly used markers are tetraspanins (CD9, CD63, CD81), endosome-derived or membrane-binding proteins (TSG101, annexins, Rabs, Syntenin, flotillines), and chaperone proteins (HSC70, HSP90). In case of PL-EVs, characteristically they are lacking CD81.
4. Determination of purity by semiquantitative comparison of cell lysates and PL-EV fractions. PL-EV fractions should not contain cellular residues like proteins from the endoplasmic reticulum (e.g. Grp94, Calnexin), Golgi apparatus (e.g. GM130), or mitochondriae (e.g. prohibitin, cytochrome C). Such markers can be used as negative markers. Additionally nucleic proteins (histones, argonaute/RISC complex) could be used as examples for negative controls.

For 3 + 4: Analytic approaches for the detection of typical EV-markers can include Western blot (WB), (high resolution-) flow cytometry, or global proteomic analysis using mass spectrometry techniques. Additional characterization of PL-EV-enriched fractions is based on the following methods:

### Proteomics

Analytic approaches for the protein-profiling of *h*PLEVs that include Western blots (WB), (high-resolution-) flow-cytometry or global proteomic analysis using mass-spectrometry are also state of the art for characterization of other EV-markers, such as immunological markers, signaling markers, cytokines and other associated bioactive protein contents.

### Microarray analysis of RNA from hPLEVs

For profiling of the *h*PLEV RNA, microarray technology can be applied. Microarrays are a well-established technique for analyzing the expression of known fragments of nucleic acids using slide or chip-based media. Microarrays are available for screening mRNA, miRNA and long non-coding RNA (IncRNA) species.

### Lipidomics

Lipids and lipid-raft-associated proteins in vesicular membranes provide extracellular vesicles with stability and structural integrity. Compared to their cells of origin, *h*PL-EVs should present a similar lipid composition.

PL-EVs may be enriched for phosphatidylserine, disaturated phosphatidylethanolamine, disaturated phosphatidylcholine, sphingomyelin, ganglioside and cholesterol. For identification of the lipid composition and ratio, mass-spectrometry, flow cytometry, or other conventional assays can be used.

### Cytokine-arrays

### Cytokine-analysis of EV-enriched fractions based on ELISA-Assays

*H*PLEV-containing fractions can be analyzed semiquantitatively for cytokine-profiles by using e.g. commercially available membrane-based cytokine-arrays. Cytokines include chemokines, tumor-necrosis-factors, interleukines, interferones and colony-stimulating factors. The technique provides a very sensitive method for detection of many different, defined proteins in parallel (e.g. 200 cytokines). Protein amounts of only a few picograms can be detected. The assay is based on membranes on which immobilized, specific primary antibodies are bound. Cytokines contained in the surrounding liquid bind to these primary antibodies during incubation. In a following reaction, a so called "sandwich-complex" is formed in which Biotin-bound secondary antibodies bind to primary antibodies. As a result, the antibody-cytokine-complex is Biotin-lebelled . HRP-bound Streptavidin or other marker-molecules can bind to Biotin and due to this make the complex detectable by chemiluminescense, calometrie or IR-light. Detection signals can be compared to signals from a known standard, in case of chemiluminescence, densiometric measurements of the signals can be performed to compare analyzed proteins.

### Functional in vitro-assays

### In vitro assays to analyze the impact of hPLEVs on immune cells

Potential immunomodulatory capabilities of enriched-EV fractions should be defined at least in one functional in *vitro*-assay using cells from the human immune system (e.g. PBMCs). The principle of such an assay is the analysis of immunomodulatory effects on immune-cells in presence or absence of *h*PLEVs. For this issue, flow cytometric-analyses are used. To induce an immune-response, cells are stimulated by adding for example PHA (phytohaemagglutinin), PMA (phorbolmyristateacetate), ionomycin, monoclonal antibodies, antigens (like candida or bacteria proteins), or other possible components even from commercial available activation kits. Also methods like mixed-lymphocyte-reaction (MLR) might be applicable. The activation might be induced unspecifically (using e.g. PHA) on all PBMCs, or specifically e.g. selectively only on T-cells or other defined PBMC-subpopulations. By the stimulation, immune-cells get activated, which can be detected amongst others by a change in the expression-profile of the cells' surface-markers. Later on an increasing proliferation-activity might also be detectable (in case of T-cells e.g.), if the stimulus is strong enough. In presence of *h*PLEV-enriched fractions, differences (such as suppression of the T cell proliferation and expression of activation markers) to the cellular control without EVs should be detectable.

### Example for such an assay: "PBMC-CFSE-PHA-Assay":

The "PBMC-CFSE-PHA-Assay" can be used for analysis of PHA-induced cellular proliferation in presence of *h*PLEV-enriched fractions. CFSE (carboxyfluorescein-succinimidylester) is a fluorescent dye that can be used for cell-tracking using a flow-cytometer for analysis. The precursor-molecule CFSE-SE (carboxyfluorescein-diacetate-succinimidylester) diffuses passively into cells, gets cleaved by intracellular enzymes and can be detected by fluorescence. Due to every cell-division, proliferating CFSE-labeled cells fade in their fluorescence-intensity for 50%.

Isolated CFSE-stained PBMCs are cultivated for 5 days in 24-well plates for suspension-cells in RPMI-media + 10% *human* AB-serum, either in presence or absence of EVs. Cell-stimulation is induced directly after starting the cultivation by 200-300 ng PHA (day 0) per well. 200 000 cells in a volume of 400 µl are cultivated per well. After 5 days, cells can be analyzed flow-cytometrically. Compared to day 0, a decrease of fluorescence indicates high proliferation-rates, stable fluorescence shows suppression of proliferation due to EVs-effects. Additionally, also expression of activation markers can be analyzed at different time points. By specific conjugated antibody-staining, cellular subpopulations of immune-cells can be discriminated and analyzed separately.

### In vitro assays to analyze the impact of hPLEVs on angiogenesis:

Angiogenesis is a fundamental process in all stages of growth and development, as well as in wound healing and tissue regeneration in ischemic vascular diseases. In angiogenetic procedure, new capillaries arise from pre-existing vasculature and the process is controlled by a sensitive balance of pro- and anti-angiogenic factors. Endothelial cells are activated in response to angiogenic stimulus such as injury, inflammation, and hypoxia.

### The tube-like formation assay

One of the most widely used *in vitro* assays to model the reorganization stage of angiogenesis is the tube formation assay. The assay measures the ability of endothelial cells to form capillary-like structures (tubes). Tube formation is typically quantified by measuring the number, length, or area of these capillary-like structures in two-dimensional microscope images of the culture dish.

### The wound healing assay

The scratch assay is used to measure cell migration *in vitro.* The basic steps involve creating a "scratch" in a cell monolayer of a homogenous population, capturing the images at the beginning and at regular intervals during cell migration to close the scratch, and comparing the images to quantify the migration rate of the cells with life imaging microscopy.
These assays can be used to study the effect of *h*PL-EVs on angiogenesis on cell-to-cell interactions, and cell migrations that may mimic cell migration during wound healing *in vivo.*

### Basic safety criteria/standards for use of hPLEVs in the clinics:

Safety, potential toxicity and immunogenicity need to be monitored for the application of *h*PLEVs in the clinics. According to the legislation for *tissues and cells*, and ATMPs ("Arzneimittel für neuartige Therapien") a platform of minimal criteria to characterize *human* cell-based medicinal products needs to be considered before use in clinical trials. In summary, it has to be addressed whether products are (a) of autologous, allogeneic or xenogeneic origin; (b) extensively or minimally manipulated *in vitro* and (c) immunologically active or neutral. In addition, (d) the proliferative capacity of cells and (e) the cell or tissue-like organization as well as the dynamic interaction amongst cells with structural components and (f) the intended use have to be defined.

Generally, the *h*PL according to the present invention originates from any thinkable *human* blood sample comprising platelets. According to a further embodiment of the present invention the *human* platelet lysate originates from platelet concentrates such as e.g. the so called platelet rich plasma (PRP). PRP is a concentrate of platelets in plasma-derived from a patient's whole blood, centrifuged to remove red-blood cells and other unwanted components. It has a greater concentration of growth factors than whole blood and has been used as a tissue injection in a variety of disciplines, including dentistry, orthopedic surgery, and sports-medicine. The platelet-concentrates might e.g. originate from buffy-coat extracted platelet concentrates or from platelet apheresis.

According to the present invention the extracellular vesicles may comprise biological factors, such as genetic material e.g. mRNA, microRNA (miRNA), small amounts of DNA, lipids and proteins including transcription factors, cytokines and growth factors.

Since the pharmaceutical preparation of the invention is deriving from platelets, it typically comprises various growth factors, particularly one or more of, preferably all of the following growth factors: PDGF, VEGF, FGF, EGF, TGF, especially TGF-β, and CTGF. The composition comprises preferably 2, 3, 4, 5 or 6 of these growth factors. Platelet-derived growth factors (PDGFs) promote cell growth and generation, repair of blood vessels and collagen-production. Vascular endothelial growth factors (VEGFs) promote growth and generation of vascular endothelial cells. Fibroblast growth factors (FGFs) promote tissue repair, cell growth, collagen-production and hyaluronic acid production. Epithelial growth factor (EGF) promotes epithelial cell growth, angiogenesis and wound-healing. Transforming growth factors (TGFs), especially TGF-β, promote growth and neogenesis of epithelial cells and wound-healing. Connective tissue growth factors (CTGFs) promote wound-repair. The pharmaceutical preparation of the invention therefore promotes the formation of new fibroblasts. These new fibroblasts start elastic and healthy, producing new collagen and less metalloproteases. The restoration of fibroblasts (the major cells in synthesizing, maintaining and providing the structural framework) results in healthier, restored skin. PDGF has also been shown to increase fibroblast motility, allowing fibroblasts to relocate to the site of administration. Natural growth factors found in the alpha-granules of platelets (such as PDGF, VEGF, FGF, EGF, and TGF) promote collagen and hyaluronic acid production, tissue repair, growth and regeneration of endothelial cells and epithelial cells, and new blood vessel formation (which restores oxygen and removes undesired molecules). All of these factors help to regenerate wrinkled and damaged extracellular matrix (ECM) back to its healthy state. Each of these growth factors plays a role within skin regeneration and restoration, both individually and additively in concert with each other. Treatments that stimulate the production of new, non-fragmented collagen will provide substantial improvement to the appearance and health of age.

According to one further embodiment preparation of the present invention, e.g. the inventive *h*PLEV-enriched fractions comprise biological factors, such as proteins, cytokines (e.g. IFN-γ, IL-8, IL-10, TGF-βI, and HLA-G, RANTES, Nap-2) and/or nucleic acids, such as, for example, microRNAs.

A preparation according to the present invention might contain cytokines with antimicrobiological properties. An inventive preparation might particularly comprise high amounts for the cytokine RANTES or the cytokine NAP-2 or for both cytokines in relative comparison to levels of other present cytokines.

The cytokines RANTES and NAP-2 have been described for their antimicrobiological properties e.g. in the article Mariani et al. (2015) (BMC Microbiology 15:149). The antimicrobiological properties of the preparation according to the present invention can be measured according to the method underlying Fig.2-Fig.7 of Mariani *et al.* and serves as reference method.

In context of the present invention it is envisaged that the preparation is suitable for e.g. intravenous administration or infusion, or for intraperitoneal injection, subcutaneous injection, intra-bone injection, intracerebroventricular injection, intra muscular injection, intraocular injection or for topical administration.

The present invention is also related to a pharmaceutical preparation comprising an enriched fraction of *human* platelet-derived extracellular vesicles obtainable by a method comprising the steps:
a) providing *human* platelet lysate from single donor donated platelets or from pooled donor-donated platelets of at least 15 donors, preferably of at least 20 or of at least 30 or of at least 40 donors.
b) enriching extracellular vesicles originating from *human* platelet lysate
c) optionally, determining an *in vitro* effect such as an immunomodulatory effect, in particular an anti-inflammatory effect and/or immune suppressive effect, of said enriched extracellular vesicles by, for example, a reduced IL-Iβ, TNF-a, T-cell proliferation, and
d) optionally, selecting those enriched extracellular vesicle fractions that exhibit the *in vitro* effect such as an immunomodulatory effect, in particular an anti-inflammatory effect and/or immune suppressive effect,
e) optionally, selecting those enriched extracellular vesicles of step b) that exhibit extracellular vesicles negative for the EV/exosome marker CD81 and positive for the EV/exosome marker CD9, and
f) optionally, admixing said enriched extracellular vesicles of step b), d) or e) with at least one suitable pharmaceutical excipient and/or carrier.

According to one embodiment of the invention *h*PL of step a might be derive from platelets that have been incubated before the lysis with one or more compounds known as thrombocyte activators from the prior art, e.g. prior art related to PRP therapy, that activate the platelets and thus improve the quality of the inventive preparation.

According to one embodiment of the invention *h*PL of step a might be derive from a Human Umbilical Cord Blood. Human Umbilical Cord Blood Platelet Lyse preparations are known from the prior art (e.g. US 8501170 B2, Parazzi, V., C. Lavazza, et al. (2015) or Forte, D., M. Ciciarello, et at. (2015).

In step a) of the inventive method as defined above *human* platelet lysate should be used that is either derived from a single donor donated platelets or from pooled donor-donated platelets of at least 15 donors, preferably of at least 20 or of at least 30 or of at least 40 donors.

If a precision medicine treatment is desired it could be of advantage to use *human* platelet lysate originating from the patient's own blood. If a general treatment is desired it is of advantage to use a pool of at least 15 donors, preferably of at least 20 or of at least 30 or of at least 40 donors in order to avoid possible individual deviations of the *human* platelet lysate in comparison with a *human* platelet lysate originating from a pool of at least 40 donors.

It might be preferred that the *h*PL from method step a) as mentioned above is provided from platelet apheresis or from buffy coat platelets, more preferred from platelet apheresis.

Immunomodulatory effects mediated by *h*PLEV-enriched fractions might be immunosuppressive effects, which are detectable with the below mentioned *in vitro-*Assays and the correspondent read-out. *h*PLEV-fractions were observed to have the ability to suppress proliferation of immune cells and therefore are immunosuppressive. Inhibitory effects on the proliferation of stimulated PBMCs can be observed in such an *in vitro* assay, also for subpopulations like CD3 positive cells (T-cells) and CD3 negative cells (including e.g. B-cells, NK-cells). Additionally, inhibitory effects on the expression of T-cell activation marker profile (downregulation of CD69 or CD25 in presence of hPLEV-fractions.

The method for producing a pharmaceutical preparation according to the present invention comprises the step of specific enrichment for EVs. As explained previously, EVs were found to be abundant in many bodily tissues and fluids and have been successfully purified using differential ultracentrifugation (Raposo, G. et al. J. Exp. Med. 1996;183(3):1161-1172). Other studies have also shown that EVs may be isolated using ultracentrifugation in a continuous density gradient of sucrose (Escola JM et al., J Biol Chem. 1998 Aug 7; 273(32):20121-7). Exosomes have also been isolated by immunoaffinity capture methods using lectins or antibodies against exosomal household markers such as CD63, CD81, EpCAM, or Rab5 (Barrès C et al., Blood. 2010 Jan 21; 115(3):696-705 and Chen, Lab Chip. 2010 Feb 21; 10(4):505-11).

Generally any suitable method for purifying and/or enrichment can be used, such as methods comprising PEG-precipitation, monolithic techniques, magnetic particles, filtration, dialysis, ultracentrifugation, ExoQuick™ (Systems Biosciences, CA, USA), chromatography or tangential flow-filtration. It is important, however, to keep in mind that depending on the isolation method, different EV-subtypes might be enriched and, even when derived from the same cell types, may differ in their functional properties
Nevertheless, a method is preferred, that comprises polyethylene glycol precipitation.

For production of a pharmaceutical preparation according to the present invention, a method is preferred, wherein EV-enriched fractions are further analyzed in microbiological tests, virulence tests, protein content, pyrogen tests, and particle size, in order to identify the most suitable fraction according to the invention.

It could be found that fractions that are enriched for *hPLEVs* were particularly useful in the methods according to the present invention, if they exhibited strong immunomodulatory effects *in vitro* activity tests.

It could further be found that fractions enriched for *h*PLEVs were particularly useful in the methods according to the present invention, if they exhibited a reduced IL-Iβ, TNF-a and/or IFN-γ cytokine response of effector cells of a donor.

Further preferred is a method according to the present invention, wherein said exosomes have a size of between about 70 to 200 nm, preferably between about 70 to 140 nm, or more preferably between about 70 to 120 nm. "About" shall mean a +/-20% deviation. Further preferred, the exosomes are positive for EV/exosome markers, and even further preferred the protein content of the pharmaceutical preparation is higher than 0,5 mg/ml and further preferred higher than 1 mg/ml (depending on the final resuspension/elution volume and initial PL-volume that is processed).

It was found that *h*PLEV enriched fractions were particularly useful in the methods according to the present invention, if they exhibited strong *in vitro* immunomodulatory effects in activity tests, and where, following the addition of said EV-fraction, a reduced IL-Iβ, TNF-a and/or IFN-γ cytokine response of effector cells of a donor could be found. ELISpot assays showed that the IL-Iβ, TNF-a and/or IFN-γ cytokine response of effector cells are impaired towards allogeneic cells in the presence of exosome containing fractions. Other methods that could be used to analyze for potential *in vitro* immunomodulatory effects, including for example Luminex, ELISA, and/or flow cytometry.

The present invention is thus based on the novel concept for an improved prevention and treatment of diseases, particularly in patients suffering from a risk of inflammatory driven diseases, neurodegenerative diseases, immune/autoimmune diseases, cardiovascular diseases, dermatologic diseases, transplant rejections, GvHD, stroke, and ischaemia and associated complications, for example, for avoiding inflammatory reactions prior or during surgery, and the prevention of inflammatory conditions and reactions of patients that are connected to a life support machine. In one embodiment, the diseases can be selected from pre- or postnatal damages of the nervous system, such as for example, brain damages related to hypoxia, inflammation, and/or ischemia. In another embodiment, the diseases can be selected from Graft versus Host-Disease, or transplant rejections following organ transplantations, respectively.

In a particularly preferred embodiment of the invention, the EV-enriched fractions derived from *h*PL that were enriched using a polyethylene glycol precipitation protocol, are prophylactically and/or therapeutically transfused into patients, in particular neonates and/or patients receiving transplants and/or patients undergoing surgery.

The pharmaceutical preparation according to the present invention preferably is enriched for EVs that comprise biological factors, such as, for example, proteins, such as anti-inflammatory cytokines, IL-10, TGF-βI, and HLA-G, and/or nucleic acids, such as, for example, miRNAs. This leads to the further advantage according to the invention that a) a multimodal (complex) intervention is performed, b) biological physiological ("self") substances are used, and c) unwanted side effects of the preparation are reduced.

The present invention constitutes a multimodal intervention. Thus not only a specific factor is used (and only a part of the cascade (or of the underlying clinical-phenotype) would be intervened with), but biologically complex and endogenous mediators and modulators are used. These components are found in every *human,* and therefore no significant adverse side- effects are expected.

Another aspect of the present invention relates to a method for production of a pharmaceutical preparation according to the present invention, comprising the following steps: a) providing *h*PL, b) enriching said *h*PL for *h*PLEVs, optionally comprising polyethylene glycol precipitation, c) determining an *in vitro* immunomodulatory effect, in particular an anti-inflammatory effect and/or immune suppressive effect, of said *h*PLEV enriched fraction by, for example, reduced IL-Iβ, TNF-a, T-cell proliferation, and/or IFN-γ cytokine response of effector cells of a donor, d) selecting those *h*PLEV enriched fractions that exhibit an immunomodulatory effect, in particular an anti-inflammatory effect and/or immune suppressive effect, and e) admixing said enriched exosomes of step d) with at least one suitable pharmaceutical excipient and/or carrier.

According to a further embodiment of the present invention, the administration is suitable e.g. for intravenous administration or infusion, or for intraperitoneal injection, subcutaneous injection, intra bone injection, intracerebroventricular injection, intramuscular injection, intraocular injection or for topical administration. Topical administration might be e.g. applied by cosmetic skin products or pavements, wound pads etc. prefabricated or pretreated or provided with an inventive preparation.

A further aspect of the present invention is a method of manufacture of a pharmaceutical preparation or a diagnostic preparation or a cosmetic preparation comprising the step of adding *human* platelet lysate or a fraction that is enriched for *human* platelet lysate derived extracellular vesicles to the pharmaceutical preparation or a diagnostic preparation or a cosmetic preparation.

The present invention includes within its scope preparations containing as an active ingredient, a therapeutically effective amount of *h*PL or a fraction of *h*PLEVs, alone or in combination with a pharmaceutical carrier or diluent. According to the desired dosage forms the skilled person might chose the suitable carrier. The dosage forms comprise e.g. tablets, granules, capsules, liquid dosage forms, gel, suppository, cream, ointment, poultice or patch. One preferred embodiment is the combination of a fraction of *h*PLEVs with a suitable polymer matrix, e.g. as described in WO2013076507. Further preferred is the intravenous application in a 0.9 % NaCl solution.

The cosmetic applications of the preparation of the present invention might be formulated with suitable excipients. Generally the *human* platelet lysate or a fraction that is enriched for *human* platelet lysate derived extracellular vesicles according to the present invention can substitute PRP where applied in cosmetics. PRP therapy has been applied to many different medical fields such as cosmetic surgery, dentistry, sports medicine and pain management. PRP has become e.g. a highly sought-after non-surgical procedure for facial and skin rejuvenation. PRP therapy is a treatment which uses blood donors own blood platelets to stimulate new cell growth, helping to improve your complexion, skin texture and to restore lost facial volume.

According to one embodiment of the present invention PRP-therapy can be substituted by a cosmetic preparation comprising *h*PL or a fraction of *h*PLEV instead of PRP. Autologuous *h*PL or a fraction of *h*PLEVs from a blood-donor himself is then re-injected into the skin to stimulate collagen and new skin cells. *H*PL or a fraction of *h*PLEVs harnesses the beneficial functions of the patients own platelets and therefore there is no risk of allergy or rejection of the treatment. *H*PL or a fraction of *h*PLEVs can also be successfully used to treat thinning hair and hair-loss particularly male pattern baldness. It might be important for a patient to start a treatment early.

A preferred embodiment of the present invention is the application of *h*PL or a fraction of *h*PLEVs in skin regeneration, such as anti-aging therapies, sun burn, allergic reactions after an insect-bite, auto-immune or allergic skin reactions, acne-inflammations. *h*PL or a fraction of *h*PLEVs might be part of cosmetic compositions for skin or hair treatment. The invention provides a regenerative therapy that directly addresses each of the problems associated with wrinkles and enhances the skin and the underlying scaffold. Treatment with a preparation described in the invention or reverses damaged skin's degenerative cycle to the healthy physiology found in normal skin. The preparation of the invention works by rebalancing cells within the connective tissues, equilibrating molecular signalling and restoring the extracellular matrix. The natural healing and tissue regeneration process leads to increased collagen synthesis, regeneration of the collagen extracellular matrix and proliferation of the fibroblasts within the matrix.

### Diagnostic use

According to the present invention based on the *h*PLEVs, *in vitro* diagnostic tests can be established for use in diagnostic applications and real-time monitoring of diseases. *H*PLEVs can be used as diagnostic biomarkers of disease through a noninvasive blood-test. The specific content of *h*PLEV preparation of an individual donor may be used as a biomarker for tumor diseases or inflammatory disease-related diseases.

To obtain a better understanding of the present invention and of its advantages, the following example is mentioned for illustrative purposes only. The example intends not to limit the scope of the present invention in any way.

### EXAMPLES

The practice of the present invention will employ, unless otherwise indicated, conventional techniques of chemistry, molecular biology, microbiology, DNA-recombination and immunology, which are within the capabilities of a person of ordinary skill in the art. Such techniques are explained in the literature. See, for example, J. Sambrook, E. F. Fritsch, and T. Maniatis, 1989, Molecular Cloning: A Laboratory Manual, Second Edition, Books 1-3, Cold Spring Harbor Laboratory Press; Ausubel, F. M. et al. (1995 and periodic supplements; Current Protocols in Molecular Biology, ch. 9, 13, and 16, John Wiley & Sons, New York, N. Y.); B. Roe, J. Crabtree, and A. Kahn, 1996, DNA Isolation and Sequencing: Essential Techniques, John Wiley & Sons; J. M. Polak and James O'D. McGee, 1990, Oligonucleotide Synthesis: A Practical Approach, Irl Press; D. M. J. Lilley and J. E. Dahlberg, 1992, Methods of Enzymology: DNA Structure Part A: Synthesis and Physical Analysis of DNA Methods in Enzymology, Academic Press; Using Antibodies: A Laboratory Manual : Portable Protocol NO. I by Edward Harlow, David Lane, Ed Harlow (1999, Cold Spring Harbor Laboratory Press, ISBN 0-87969-544-7); Antibodies : A Laboratory Manual by Ed Harlow (Editor), David Lane (Editor) (1988, Cold Spring Harbor Laboratory Press, ISBN 0-87969-314-2), 1855; and Lab Ref: A Handbook of Recipes, Reagents, and Other Reference Tools for Use at the Bench, Edited Jane Roskams and Linda Rodgers, 2002, Cold Spring Harbor Laboratory, ISBN 0-87969-630-3.

### Example 1: Method-examples of preparation of platelet rich plasma

### 1.1. Platelet concentrate (PRP-PC)

450 ml of whole blood are collected in a 450-ml triple bag containing CPDA1 anticoagulant (TERUMO PENPOL, Ltd. Puliyarakonam, Trivandrum, India). Platelet rich plasma is separated from whole blood by light spin centrifugation by Heraeus 6000i, Germany refrigerated centrifuge at 1750 rpm for 11 minutes at 21°C, with acceleration and deceleration curves of 5 and 4 respectively and the platelets are concentrated by heavy spin centrifugation at 3940 rpm for 5 minute at 21°C, with acceleration and deceleration curves of 9 and 5 respectively with subsequent removal of supernatant plasma. The platelet concentrate bag is left stationary with the label side down at room temperature for approximately 1 hour. The platelet poor plasma is frozen promptly and stored as fresh frozen plasma (FFP) at or below - 30°C for one year.

### Method for preparation of buffy coat - platelet concentrate (BC-PC)

450 ml of whole blood is collected in a 450-ml quadruple bag containing 63 ml of CPD anticoagulant, with additive solution (SAGM). (TERUMO PENPOL, Ltd.,Puliyarakonam, Trivandrum, India). The whole blood is first subjected to "hard spin" centrifugation at 3940 rpm for 5 minutes at 21°C with acceleration and deceleration curves of 9 and 4 respectively. Whole blood is separated into different components according to their specific gravity.
- The top layer - platelet poor supernatant plasma (150-200 ml).
- Middle layer - buffy coat, containing approximately 90% of platelets, 70% of WBCs and 10% of red cells.
- Bottom layer - packed red cells.
Platelet poor supernatant is expressed into one satellite bag and buffy coat into another satellite bag. About 20-30 ml of plasma is returned to buffy coat with the aim of cleaning the tubing from residual cells and obtaining an appropriate amount of plasma in the BC. The SAGM solution is added to the red cells. The bags containing red cells and plasma are then removed. Red cells are placed at 4°C in cold room and platelet poor plasma into a -40°C deep freezer as fresh frozen plasma (FFP). The buffy coat is gently mixed with the plasma and again subjected to 'light spin" centrifugation at 1,100 rpm for 6 minutes at 21°C, with acceleration and deceleration curves of 5 and 4 respectively, along with one empty satellite bag. The supernatant, platelet rich plasma is expressed into empty platelet storage bag and then tubing was sealed. The bag with residual WBCs and red cells was discarded.

### 1.2. Single donor platelets (SDP)-apheresis-PC

The automated cell separator equipment may be of intermittent flow or continuous flow cell technique, using single or double venous access. Continuous flow, double venous access, automated cell separator - CS3000 ® plus, Baxter, Fenwal division, Deerfield, 14 60015, USA can be used.
1. Written consent of the donor is taken after explaining the procedure in detail, time taken and about possible hazards and benefits.
2. Venous access is an important consideration in apheresis donors and veins are examined at the time of selection because of the following reasons -
   - Long duration of procedure
   - Prolonged flow rate
   - Frequent need for two venipunctures with continuous flow equipment.
3. Age of the donors is documented.
4. Prior to the apheresis procedure, ABO / Rh typing and testing for infectious disease markers (HIV, HBV, HCV, VDRL and malaria) of plateletpheresis-donors are done.
5. Donors who have taken aspirin or other NSAIDS, which are likely to affect the platelet function, are deferred.
6. Those donors who have platelet count > 1.5 × 10⁵/µl were taken for plateletpheresis.

### 1.3. Procedure

The procedure is performed in a closed system. A disposable kit is installed to the continuous flow separator, then the machine is primed. The donor is prepared by cleaning of two venipuncture sites at the antecubital area of both arms by betadine and spirit phlebotomy is done with minimal trauma to the donor. During the procedure, the blood is anti-coagulated at the point of withdrawal in a controlled manner, and the ratio of whole blood and anticoagulant (ACD) is maintained at 9:1 to 11:1. The anticoagulated blood is pumped into a spinning separation container. Red cells are packed by centrifugal force towards the outer edges of the container, and then the red cells exit the separation container. The lower density components, such as plasma, platelets, or WBCs are removed by plasma pump and enter the spinning collection container where platelets are packed by centrifugal force towards the outer edges of the container. The separated platelets remain packed in the container, while other constituents of blood are returned to the donor. At the end of the collection procedure, the platelet collection bag is shaken vigorously to detach the platelets from the wall of the bag and kept for 1 hour at room temperature to make it an even suspension. This whole procedure requires 1.5-2.5 hours. The final volume of the apheresis-PC ranged from 200-300 ml.

### Example 2: Production of human thrombocyte lysates (hPL)

Production of *human* thrombocyte lysate (= *human* platelet lysate (*h*PL)) is based on further processing of expired *human* thrombocyte concentrates (= *human* platelet concentrates (*h*PC/*h*TC). To gain *h*TCs usually two different proceeding methods are used. One is defined by the use of pooled buffy coat products deriving from whole blood donations of several donors, in the other apherese-concentrates are used. In this technique a donor is connected to an extracorporal cell-separator and thrombocytes are filtered out, while other blood components like erythrocytes, leucocytes and plasma are given back to the donor. In both cases the resulting blood products are leucocyte-depleted TCs. These products contain living thrombocytes for at least 4-5 days and can be used e.g. to replace lacking thrombocytes in patients with thrombocytopenia.
After date of expiry, excess products can be used for production of *h*PL. By freezing and thawing-cycles (-20 °C and RT) platelets are broken up/cracked and release their inner contents into the surrounding liquid (plasma). Different protocols exist including centrifugation steps between 3200 and 10 000 g for about 1 hour. Lysed cells, cellular fragments and other debris is/are depleted by centrifugation. The result is a clear, vicious, yellow liquid, consisting of the platelet lysates and plasma. To sterilize the product after proceeding, 200 nm filters can be used optionally in addition. In parallel particles bigger than 200 nm are removed, including extracellular vesicles, which are not at the size of exosomes.

### Example 3: Isolation of peripheral mononuclear cells (PBMCs) using Ficoll-density gradient centrifugation and cultivation of cells in vitro

Peripheral mononuclear cells are gained from buffy-coat products deriving from a full-blood donation. Isolation of PBMCs was performed by using Ficoll-density gradient centrifugation according to the following description:
1. About 100 ml of a buffy coat product is distributed on three 50 ml polypropylene tubes to equal parts. If necessary, missing volume was replaced by PBS (phosphate-buffered saline).
2. Three additional 50 ml PP-tubes is filled up with 10 ml of Ficoll.
3. By pouring carefully 35 ml of blood onto 10 ml of Ficoll, two separated layers are formed.
4. Density-gradient centrifugation is performed at 900 g for 20 minutes at temperatures of 10 °C (break set on 0 or 1).
5. After formation of the gradient, the PBMC-containing interphase is transferred into a new 50 ml PP-tube.
6. Collected fraction with PBMCs is filled up to 50 ml of volume using PBS.
7. For depletion of platelets centrifugation at 650 g for 5 minutes is performed.
8. Supernatants containing platelets are discarded.
9. For lysis of erythrocytes, cells are resuspended in 20-25 ml of lysing-buffer followed by 7 minutes of incubation at 4°C.
10. Lysing reaction is stopped by filling up PBS to a volume of 50 ml by PBS.
11. Depletion of lysed fragments by centrifugation at 900 g for 5 min.
12. Supernatant is discarded and cells resuspended in 50 ml PBS.
13. Cells are counted manually using a Neubauer-chamber or alternatively automatically e.g. by using the Sysmex-haemocytometer.
14. PBMCs are resuspended in culture-media (RPMI, 10% of heat inactivated *h*AB-Serum, 1% PSG (penicillin/streptomycin/L-Glutamin) and set on an appropriate concentration according to following *in vitro*-assays.

### Example 4: Analyses and characterization of extracellular vesicle-preparations on a molecular level

### 1. Determination of total protein concentration using a BCA-Assay (or alternative standard methods like Bradford-Assay)

Total protein concentrations of EV-enriched fractions can be determined by using standard methods. A variety of commercially kits and agents is available for this purpose. For example the BCA Protein Assay Kit from Thermo Scientific was used. Two molecules of bicinchoninacid (BCA) form a chelate complex together with one copper ion (1+). The copper reduction is caused by the presence of proteins in an alcalic environment. Formation of chelate complexes corresponds to a colour change of the analyzed liquids from green to purple. The intensity of the colour change can be measured photometrically at absorption of 562 nm. Compared to known values of calibration values of different BSA-concentrations, the protein-concentration of the EV fractions can be determined.

### 2. Determination of mean particle size [nm] and size distribution [curves] and particle numbers (using NTA-platforms like Nanosite or Zetaview)

For characterization of extracellular nanovesicles, the *Nanoparticle Tracking Analysis* (NTA) was used. This physical technique is suitable for tracking particles from a smaller size than the wave length of light. The method is based on the induction of an electric field, in which the particles start to move. Due to this movement, their Brownian motion can be tracked during their diffusion through the analysis cuvette. Size and size-distribution of particles in a fraction can be defined and also values for particle concentrations are given. The tracking analysis of the Brownian motion can be followed on a screen connected to the video microscope. Data are converted digitally by the software into data. Determination of size is calculated by the particles diffusion constant and converted into hydrodynamic particle sizes. Particle concentrations are deduced from the analysis of the video sections and are related to the measured amount of scatter light.

### Isolation Methods

Isolation methods can be based on ultracentrifugation (differential centrifugation), on size-exclusion chromatography (Izon and Exospin columns), on polymer-based precipitation (PEG 1000, PEG 6000, PEG 8000 EV, Exoquick-Qiagen), on membrane affinity (Exoeasy-Qiagen), and flow filtration. There is no standardized state-of-the-art technology to isolate EVs, for either therapeutic application or basic research. The criteria for the selection of the purification method are the initial volume of platelet lysate that has to be processed as well as high purity and recovery of the enriched PL-EV fractions.

Selection of the purification method needs to be standardized with regard to the reproducibility, purity, impurities, and maintenance of *h*PLEVs' functional properties. Applied methods should be evaluated in the context of their scalability and reproducibility. Methods yielding the highest *h*PLEV purity will not necessarily be optimal for recovering the therapeutically most effective EV fractions due to the fact that components attached to the *h*PLEV surface or co-isolated non-*h*PLEV associated co-factors might be lost during purification.

### EV storage

For storage of EVs no standardized protocol is currently available. Storage conditions must be validated because they may affect the EVs' stability. A number of common used solvents and buffers range from sodium chloride to PBS, TRIS-HCI, HEPES and glycerol.

### Example 5: Principles of Centrifugation

Centrifugation is used for separating components, cells and for isolation of cell organelles. It is based on the movement of particles in a liquid caused by centrifugal force. Main component of this technique is the rotor. Different types exist like fixed-angle rotors, vertical rotors or swing rotors. Ultracentrifuges belong to the group of high-speed centrifuges. To avoid the development of frictional heat due to aerodynamic drag, a vacuum is set up. According to the physical principle, separation of components occurs due to size and density. Particles are accelerated by centrifugal forces in outward-direction. This acceleration depends on the angular velocity of a particle and its distance to the rotation axis. Acceleration refers to the force of gravity g.
By the Svedberg-equation, sedimentation-speed of spherical particles in a vicious fluid is described. S-value (Svedberg-units) from biologic material: The coefficient of sedimentation s is defined as the sedimentation-speed that is achieved under special geometric conditions in a centrifugal field. The unit of the sedimentation-coefficient s is defined as S-value. Various techniques of centrifugation exist: differential centrifugation, zonal centrifugation, isopycnic centrifugation, density gradient centrifugation.

### Differential centrifugation:

Differential centrifugation is based on different sedimentation-speeds of particles.
It is used for enrichment of particles and for gaining a higher concentration of particles in a reduced volume. Fixed-angle rotors are used.
Therefore it is required, that sedimentation-speeds of centrifuged particles differ enough from each other.

Related to cells and their components following differences exist, that allow separation:
Complete cells sediment first (1 000 g, 2 min), followed by larger-sized cellular components of high weight like nuclei (1 000 g, 5-10 min). Nuclear membranes and plasma membranes sediment afterwards (1 500 g, 15 min), followed by the golgi apparatus (2 000 g, 20 min), mitochondriae, lysosomes and peroxysomes (10 000 g, 25 min). Microsomes sediment at 100 000 g, 60 min or longer. To these belong also EVs including exosomes. They are found in the final pellet.

### Purity of fractions gained by differential centrifugation:

Described components cannot be separated and purified to 100%. Sediments consisting out of quickly sedimenting particles will always include slowly sedimenting particles, which were placed nearby the bottom of the centrifuge tube. Due to this contamination, no complete purity can be achieved.

### Differential centrifugation used for enrichment of EVs/Exosomes:

In a first centrifugation step, EV-containing liquids (like cell culture supernatants, diluted Plasma-containing liquids or diluted hPL) are centrifuged at 2000 g for 15 min. Cells, dead cells, cellular debris (nuclei, nuclear membranes, plasma membranes, Golgi apparatus) pellet at the bottom and can be removed. In a second centrifugation step at 10 000g for 45 min at 4°C, the supernatants of step 1 are depleted from mitochondriae, lysosomes and peroxysomes. Microsomes like EVs including exosomes stay in the supernatant and can finally be pelleted by ultracentrifugation (110 000 g, 1-2 hours).

### PEG-Precipitation

For the polyethyleneglycol-precipitation, PEG 6000 can be used. The substance is a polymer deriving from ethylene-glycol and is water-soluble, inert and not toxic. PEG can be used to precipitate high-molecular substances like proteins (also virus-particles and EVs). In presence of PEG proteins precipitate while low-molecular substances stay soluble. Depending on the chosen precipitation-conditions, the border to define high-molecular and low-molecular substances can vary to a certain degree (molecular-weight of PEG, PEG-concentration and precipitation temperature). If hydrophilic, uncharged PEG and proteins are mixed together in aqueous solutions, there is concurrence occurring for the hydration water of the proteins. If a defined PEG-concentration is reached, proteins precipitate in a reversible way. This precipitation represents a very gentle way of precipitation (first described: Polson et al., 1964).

### PEG-precipitation of EVs:

EV-containing liquids, diluted e.g. in 0,9% NaCl, are incubated in presence of 10% v/v PEG 6000 overnight (16 h, 4°C) to precipitate EVs. After incubation, precipitated particles are pelleted at 1 500 g for 30 min (4°C). Supernatants are discarded. Pellets are resuspended e.g. in 0,9% NaCl and brought into ultracentrifugation (110 000 g, ca. 2 h). This step can alternatively be repeated as an additional washing step.

In a preferred embodiment it is provided for a method for preventing and/or the treatment of a disease selected from the group consisting of regenerative diseases, inflammatory driven diseases, neurodegenerative diseases, immune/autoimmune diseases, cardiovascular diseases, dermatologic diseases, infectious diseases, transplant rejections, stroke, ischaemia or Graft-versus-Host Disease, comprising administering to said patient an effective amount of a pharmaceutical preparation according to any of claims 1 to 13.

Peferably, this method is a method, wherein said administration is suitable for intravenous administration or infusion, or for intraperitoneal injection, subcutaneous injection, intra bone injection, intracerebroventricular injection, intra muscular injection, intraocular injection or for topical administration.

Preferably, it is provided for a method of manufacture of a pharmaceutical preparation or a diagnostic preparation or a cosmetic preparation comprising the step of adding human platelet lysate or a fraction that is enriched for *human* platelet lysate derived extracellular vesicles to the pharmaceutical preparation or a diagnostic preparation or a cosmetic preparation.

### REFERENCES

1. Thomas Lener et al. in ISEV position paper in the Journal of Extracellular Vesicles 2015, 4: 30087
2. Torreggiani et al. (European Cells and Materials Vol.28, 2014 137-151
3. Foster TE, Puskas BL, Mandelbaum BR, Gerhardt MB, Rodeo SA (2009). Am J Sports Med 37 (11): 2259-72)
4. Koliha, Nina et al. Journal of Extracellular Vesicles, [S.I.], Feb. 2016.
5. Craig N. Morrell et al., May 1, 2014; Blood: 123 (18)
6. Kuffler DP et al. in Mol Neurobiol. 2015 Oct;52(2):990-1014. doi: 10.1007/s12035-015-9251-x. Epub 2015 Jun 6
7. Raposo, G. et al. J. Exp. Med. 1996;183(3):1161-1172
8. Escola JM et al., J Biol Chem. 1998 Aug 7; 273(32):20121-7).
9. Barrès C. et al., Blood. 2010 Jan 21; 115(3):696-705
10. Chen, Lab Chip. 2010 Feb 21; 10(4):505-11).
11. Mariani et al. BMC Microbiology (2015) 15:149
12. Eppley, B. L., W. S. Pietrzak, et al. (2006) Plast Reconstr Surg 118(6): 147e-159e.
13. Mishra, A., K. Harmon, et al. (2012) Curr Pharm Biotechnol 13(7): 1185-1195 Carlson, N. E. and R. B. Roach, Jr. (2002) J Am Dent Assoc 133(10): 1383-1386.
14. Fontana, F., M. Mori, et al. (2016) ACS Appl Mater Interfaces 8(1): 988-996.
15. Naaijkens, B. A., H. W. Niessen, et al. (2012) Cell Tissue Res 348(1): 119-130.
16. Govindasamy, V., V. S. Ronald, et al. (2011) Cytotherapy 13(10): 1221-1233.
17. Parazzi, V., C. Lavazza, et al. (2015). "Extensive Characterization of Platelet Gel Releasate From Cord Blood in Regenerative Medicine." Cell Transplant 24(12): 2573-2584.
18. Forte, D., M. Ciciarello, et al. (2015). "Human cord blood-derived platelet lysate enhances the therapeutic activity of adipose-derived mesenchymal stromal cells isolated from Crohn's disease patients in a mouse model of colitis." Stem Cell Res Ther 6: 170.

## Claims

1. Pharmaceutical preparation comprising a fraction that is enriched for *human* platelet lysate derived extracellular vesicles for use in medicine.

2. Pharmaceutical preparation comprising a fraction that is enriched for human platelet lysate derived extracellular vesicles for use in the prevention and/or treatment of inflammatory driven diseases, neurodegenerative diseases, immune/autoimmune diseases, cardiovascular diseases, dermatologic diseases, infectious diseases, transplant rejections, stroke, ischemia or Graft-versus-Host Disease.

3. Preparation for use according to claim 1 or 2, wherein the preparation is cell-free.

4. Preparation for use according to any of claims 1 to 3, wherein the enriched fraction of *human* platelet lysate derived extracellular vesicles is the essential pharmaceutically active ingredient in the preparation.

5. Preparation for use according to any of claims 1 to 4, wherein the extracellular vesicles of the enriched fraction have a size of between 10-1000 nm, particularly a size of between 50 to 200 nm preferably between 70 to 140 nm.

6. Preparation for use according to any of claims 1 to 5, wherein the extracellular vesicles of the enriched fraction are positive for at least one of the cellular exosome markers consisting of the group: CD9, CD41a, CD41b, CD42b, CD61, CD 62P, CD63 and Syntenin.

7. Preparation for use according to any of claims 1 to 6, wherein the extracellular vesicles of the enriched fraction are negative for at least one of the cellular exosome markers consisting of the group CD81, CD3, CD4, CD19, CD20, CD2, CD8, CD11a and CD25.

8. Preparation for use according to any of claims 1 to 7 for antimicrobiological applications, wherein the extracellular vesicles of the enriched fraction are positive for the cytokine RANTES or the cytokine NAP-2 or for both.

9. Preparation for use according to any of claims 1 to 8, wherein the protein content of the pharmaceutical preparation is higher than 0.5 mg/ml.

10. Preparation for use according to any of claims 1 to 9, wherein the *human* platelet lysate originates from single or pooled donor-donated platelets.

11. Preparation for use according to any of claims 1 to 10, wherein the *human* platelet lysate originates from buffy-coat extracted platelet concentrates or from platelet apheresis.

12. Preparation for use according to any of claims 1 to 11, wherein the extracellular vesicles comprise biological factors, such as genetic material such as mRNA, microRNA (miRNA), small amounts of DNA, lipids and proteins including transcription factors, cytokines, growth factors.

13. Preparation for use according to any of claims 1 to 12, wherein said preparation comprises a pharmaceutical acceptable carrier, preferably a pharmaceutical acceptable polymer.

14. Preparation for use according to any of claims 1 to 13, wherein said preparation is suitable for intravenous administration or infusion, for intraperitoneal injection, subcutaneous injection, intra bone injection, intracerebroventricular injection, intra muscular injection, intraocular injection or for topical administration.

## Patentansprüche

1. Pharmazeutisches Präparat mit einer Fraktion, die mit von menschlichem Blutplättchenlysat abgeleiteten extrazellulären Vesikeln angereichert ist, zur Verwendung in der Medizin.

2. Pharmazeutisches Präparat mit einer Fraktion, die mit von menschlichem Blutplättchenlysat abgeleiteten extrazellulären Vesikeln angereichert ist, zur Verwendung bei der Vorbeugung und/oder Behandlung von entzündlich bedingten Erkrankungen, neurodegenerativen Erkrankungen, Immun-/Autoimmunerkrankungen, Herz-Kreislauf-Erkrankungen, dermatologischen Erkrankungen, Infektionskrankheiten, Transplantatabstoßungen, Schlaganfall, Ischämie oder Graft-versus-Host-Erkrankung.

3. Präparat zur Verwendung nach Anspruch 1 oder 2, wobei das Präparat zellfrei ist.

4. Präparat zur Verwendung nach einem der Ansprüche 1 bis 3, wobei die angereicherte Fraktion der von menschlichem Blutplättchenlysat abgeleiteten extrazellulären Vesikel der wesentliche pharmazeutisch aktive Bestandteil in dem Präparat ist.

5. Präparat zur Verwendung nach einem der Ansprüche 1 bis 4, wobei die extrazellulären Vesikel der angereicherten Fraktion eine Größe zwischen 10 und 1000 nm, insbesondere eine Größe zwischen 50 und 200 nm, vorzugsweise zwischen 70 und 140 nm haben.

6. Präparat zur Verwendung nach einem der Ansprüche 1 bis 5, wobei die extrazellulären Vesikel der angereicherten Fraktion positiv für mindestens einen der zellulären Exosomenmarker sind, die aus folgender Gruppe bestehen: CD9, CD41a, CD41b, CD42b, CD61, CD 62P, CD63 und Syntenin.

7. Präparat zur Verwendung nach einem der Ansprüche 1 bis 6, wobei die extrazellulären Vesikel der angereicherten Fraktion negativ für mindestens einen der zellulären Exosomenmarker sind, die aus folgender Gruppe bestehen: CD81, CD3, CD4, CD19, CD20, CD2, CD8, CD11a und CD25.

8. Präparat zur Verwendung nach einem der Ansprüche 1 bis 7 für antimikrobiologische Anwendungen, wobei die extrazellulären Vesikel der angereicherten Fraktion für das Zytokin RANTES oder das Zytokin NAP-2 oder für beide positiv sind.

9. Präparat zur Verwendung nach einem der Ansprüche 1 bis 8, wobei der Proteingehalt des pharmazeutischen Präparats höher als 0,5 mg/ml ist.

10. Präparat zur Verwendung nach einem der Ansprüche 1 bis 9, wobei das menschliche Blutplättchenlysat von Blutplättchen stammt, die von einem einzelnen Blutspender oder einem Pool von Blutspendern gespendet wurden.

11. Präparat zur Verwendung nach einem der Ansprüche 1 bis 10, wobei das menschliche Blutplättchenlysat von Blutplättchen-Konzentraten, die über eine Leukozytenmanschette extrahiert werden, oder aus der Blutplättchen-Apherese stammt.

12. Präparat zur Verwendung nach einem der Ansprüche 1 bis 11, wobei die extrazellulären Vesikel biologische Faktoren wie genetisches Material wie mRNA, microRNA (miRNA), kleine Mengen an DNA, Lipide und Proteine einschließlich Transkriptionsfaktoren, Zytokine und Wachstumsfaktoren umfassen.

13. Präparat zur Verwendung nach einem der Ansprüche 1 bis 12, wobei das Präparat einen pharmazeutisch annehmbaren Träger, vorzugsweise ein pharmazeutisch annehmbares Polymer umfasst.

14. Präparat zur Verwendung nach einem der Ansprüche 1 bis 13, wobei das Präparat geeignet ist für eine intravenöse Verabreichung oder Infusion, für eine intraperitoneale Injektion, subkutane Injektion, Injektion in den Knochen, intrazerebroventrikuläre Injektion, intramuskuläre Injektion, intraokulare Injektion oder für eine topische Verabreichung.

## Revendications

1. Préparation pharmaceutique comprenant une fraction qui est enrichie de lysat de plaquettes humaines dérivées de vésicules extracellulaires pour utilisation en médecine.

2. Préparation pharmaceutique comprenant une fraction qui est enrichie de lysat de plaquettes humaines dérivées de vésicules extracellulaires utilisées dans la prévention et/ou le traitement de maladies inflammatoires, de maladies neurodégénératives, de maladies immunes/auto-immunes, de maladies cardiovasculaires, de maladies dermatologiques, de maladies infectieuses, de rejets de greffe, AVC, d'ischémie ou de réactions de greffon contre l'hôte.

3. Préparation à utiliser selon la revendication 1 ou 2, dans laquelle la préparation est acellulaire.

4. Préparation à utiliser selon l'une quelconque des revendications 1 à 3, dans laquelle la fraction enrichie de lysat de plaquettes humaines dérivées de vésicules extracellulaires est l'ingrédient actif pharmaceutique essentiel dans la préparation.

5. Préparation à utiliser selon l'une quelconque des revendications 1 à 4, dans laquelle les vésicules extracellulaires de la fraction enrichie ont une taille comprise entre 10-1000 nm, notamment une taille comprise entre 50 à 200 nm, de préférence entre 70 à 140 nm.

6. Préparation à utiliser selon l'une quelconque des revendications 1 à 5, dans laquelle les vésicules extracellulaires de la fraction enrichie sont positives pour au moins un des marqueurs exosomes cellulaires dans le groupe : CD9, CD41a, CD41b, CD42b, CD61, CD 62P, CD63 et la synténine.

7. Préparation à utiliser selon l'une quelconque des revendications 1 à 6, dans laquelle les vésicules extracellulaires de la fraction enrichie sont négatives pour au moins un des marqueurs exosomes cellulaires dans le groupe CD81, CD3, CD4, CD19, CD20, CD2, CD8, CD11a et CD25.

8. Préparation à utiliser selon l'une quelconque des revendications 1 à 7 pour des applications antimicrobiologiques, dans laquelle les vésicules extracellulaires de la fraction enrichie sont positives pour la cytokine RANTES ou la cytokine NAP-2 ou pour les deux.

9. Préparation à utiliser selon l'une quelconque des revendications 1 à 8, dans laquelle la protéine contenue dans la préparation pharmaceutique est supérieure à 0,5 mg/ml.

10. Préparation à utiliser selon l'une quelconque des revendications 1 à 9, dans laquelle le lysat de plaquettes humaines a pour origine de plaquettes données par un donneur unique ou par un ensemble de donneurs.

11. Préparation à utiliser selon l'une quelconque des revendications 1 à 10, dans laquelle le lysat de plaquettes humaines a pour origine des concentrés de plaquettes extraites de la couche leucoplaquettaire ou d'une aphérèse de plaquettes.

12. Préparation à utiliser selon l'une quelconque des revendications 1 à 11, dans laquelle les vésicules extracellulaires comprennent des facteurs biologiques, tels que le matériau génétique tel que de l'ARNm, du microARN (miRNA en anglais), des petites quantités d'ADN, des lipides et des protéines comprenant des facteurs de transcription, des cytokines, des facteurs de croissance.

13. Préparation à utiliser selon l'une quelconque des revendications 1 à 12, dans laquelle ladite préparation comprend un porteur pharmaceutique acceptable, de préférence un polymère pharmaceutique acceptable.

14. Préparation à utiliser selon l'une quelconque des revendications 1 à 13, dans laquelle ladite préparation est adaptée pour une administration intraveineuse ou une perfusion, pour une injection intrapéritonéale, une injection sous-cutanée, une injection intra-osseuse, une injection intraventriculaire, une injection intra-musculaire, une injection intraoculaire ou pour une administration topique.
